(19) Europäisches Patentamt
European Patent Office
Office européen des brevets

(11) Publication number: **0 318 734 A1**

(12) **EUROPEAN PATENT APPLICATION**

(21) Application number: **88118706.6**

(51) Int. Cl.[4]: **G01N 33/569 , G01N 33/535**

(22) Date of filing: **10.11.88**

(30) Priority: **30.11.87 JP 302524/87**

(43) Date of publication of application: **07.06.89 Bulletin 89/23**

(84) Designated Contracting States: **AT BE CH DE ES FR GB GR IT LI LU NL SE**

(71) Applicant: **INSTITUT VIRION AG**
**Weingartenstrasse 9**
**CH-8803 Rüschlikon(CH)**

(72) Inventor: **Takeda, Yoshifumi**
**1-13-19-105, Yohga Setagaya-ku**
**Tokyo 158(JP)**
Inventor: **Oku, Yuichi**
**3-15-22-203, Shirokanedai Minato-ku**
**Tokyo 108(JP)**

(74) Representative: **Scheidegger, Zwicky, Werner & Co.**
**Stampfenbachstrasse 48 Postfach**
**CH-8023 Zürich(CH)**

(54) **A highly sensitive method to detect bacteria by using antibody against bacterial cells.**

(57) The method for detecting bacteria, in which seleceted antibodies are used against supposed bacterial antigens, conjugated with peroxidase by using a succimide of the general formula

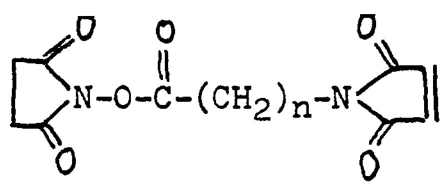

wherein n is 3, 4 or 5.

EP 0 318 734 A1

Xerox Copy Centre

# A HIGHLY SENSITIVE METHOD TO DETECT BACTERIA BY USING ANTIBODY AGAINST BACTERIAL CELLS

## BACKGROUND OF THE INVENTION

### 1. FIELD OF THE INVENTION

The present invention relates to a highly sensitive, simple and rapid method to detect and identify bacteria by using the specified reagents for enzymelinked immunosorbent assay.

### 2. DESCRIPTION OF TECHNIQUES THAT HAVE BEEN IN PRACTICAL USE (PRIOR ART)

In bacterial infections, it is necessary to identify the causative bacteria to treat patients. For this purpose, the currently adopted method is first to diagnose patients clinically by doctors and then to select possible pathogens to be cultured by using various media. For instance, in diarrheal diseases, stools are collected and streaked on various media including selective media in order to isolate a possible specific pathogen among many other non-pathogenic bacteria. This method is based on having a pure culture, thus it is difficult to isolate the pathogen within a short period of time. Moreover, there are several other disadvantages in this method. First of all, this method cannot detect bacteria that are incapable or difficult to grow on routinely available media. Secondly, bacteria that can grow but take a long time (for example more than 3 days) cannot be identified rapidly, thus the information obtained cannot be used for the treatment. Thirdly, when the patients have taken antibiotics before collecting the specimen, it is quite difficult or impossible to obtain the growth of the causative bacteria.

To overcome these disadvantages, immunological methods have been developed. This method is to detect the specific antigens derived from the causative bacteria in tissues or fluids of patients by using the antibody against the specific antigen.

Antigen-antibody reactions have some disadvantages and advantages as well. Some of the disadvantages are: (1) the reaction is so specific that the selection of the antibody to be used in the reaction should be correct, and (2) sensitivity and accuracy of the reaction varied quite considerably with reagents used and with various antigen-antibody reactions applied. On the other hand, some of the advantages are: (1) the reaction is quite specific, and (2) the results are obtained within a short time. Thus, various methods by using various antigen-antibody reactions, such as precipitin reaction, agglutination and complement fixation reaction, have been developed. Also various enzymes and various fluorescent materials have been used to visualize the reactions.

Enzyme-linked immunosorbent assay (ELISA) has been widely used because of its accuracy, high sensitivity and simplicity. There are two methods, that is, competitive ELISA and sandwich ELISA. Especially the latter method has been used widely.

The principle of the sandwich ELISA is as follows. First an insoluble antibody is prepared by adsorbing the antibody to an inactive and insoluble solid phase. Then the antigen in the sample is coupled to the insoluble antibody by antigen-antibody reaction. To this antigen- antibody complex, enzymelinked antibody is added and the antigen is sandwiched by two types of antibody. The amount of the enzymelinked antibody is measured by the enzymatic reaction, thus the amount of the antigen in the sample is measured.

To prepare enzyme-antibody conjugate to be used for the sandwich ELISA, several reagents, such as glutaladehyde (Immunochemistry, 6: 43, 1969, Immunochemistry, 8: 871, 1971), periodate (J. Histochem. Cytochem., 22: 1084, 1974), pyridyl disulfide (Biochem. J., 173: 723, 1978) and maleimide (J. Biochem., 78: 235, 1975), have been used. It has been known that to prepare more sensitive conjugate maleimide is better than glutaraldehyde and periodate (J. Applied Biochemistry, 4: 41, 1982). Imagawa et al. (J. Applied Biochemistry, 4: 41 1982) also reported that antibody against human ferritin conjugated with peroxidase by maleimide was a better reagent than other chemicals to measure human ferritin by ELISA. There are several patents applied (58-149700 and 59-176675) in which conjugates by maleimide is to be used in ELISA to measure human immunoglobulin G, $\alpha$-fetoprotein, several hormones and drugs. However, these patents applied have neither described on the usefulness for sensitive, rapid and simple detection of bacteria nor suggested that the method is more useful or valuable than the previously reported methods to detect bacteria.

## SUMMARY OF THE INVENTION

It is an object of the present invention to provide new and useful method permitting a highly sensitive, simple and very rapid method to detect and identify bacteria by using antibodies against bacterial antigens conjugated with peroxidase by using N-(maleimidoalkanoyloxy)-succimide of the general formula:

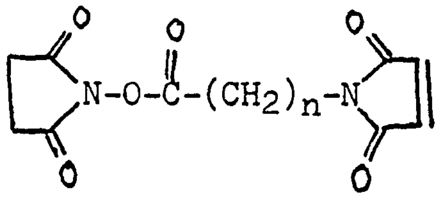

(number of n is 3 to 5)

## DETAILED DESCRIPTION OF PREFERRED EMBODIMENTS OF THE INVENTION

To detect and identify the causative bacteria of the infections, a highly sensitive, rapid and simple method is necessary. The invention provides such a method. The method developed can apply not only to bacterial culture but also to specimens, such as diarrheal stools, so that rapid diagnosis of the infection can be achieved.

### Description of embodiment

(i) Specimens to be used

Not only the bacteria cultured in various media but also the specimens from patients, such as diarrheal stools.

(ii) Preparation of the antibody

1. Monoclonal antibody against bacterial cells and other bacterial antigens
2. Polyclonal antibody against bacterial cells and other bacterial antigens

(iii) Reagents to be used in the inventive method

Immunologically active complex: the antibody against bacterial antigens conjugated with peroxidase by using N-(maleimidoalkonoyloxy)-succimide.

N-(maleimidoalkanoyloxy)-succimide has been reported in J. Biochem., 79, 223, 1979, and in the Japanese patent 52-85163 and 52-85164. The compound can be synthesized by the method described in the above reports. In the general formula of N-(maleimidoalkanoyloxy)- succimide described above, the compound with n = 3 is N-(γ-maleimidobutyloxy)-succimide, that with n = 4 is N-(δ-maleimidopentyloxy)-succimide, and that with n = 5 is N-(ε-maleimidocaproyloxy) -succimide (succidyl-6-maleimidohexanoate). Among these compounds, the compound with n = 5 is the most appropriate to prepare the most sinsitive reagent to be used in the ELISA.

To conjugate the antibody with the peroxidase by succidyl-6-maleimidohexanoate (n = 5), the first reaction is to react the -NH base of the enzyme with the maleimide and obtain the following.

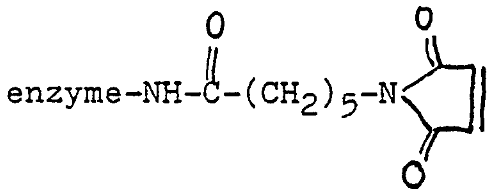

The second reaction is to react this compound with -SH base of the antibody and obtain the following conjugate.

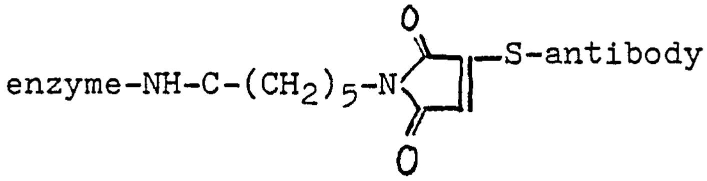

The compounds to be conjugated to the enzyme are the various antibodies against various bacterial antigens. The enzymes to be used are peroxidases from various sources, such as horseradish, pineapple, sweet potato, broad bean, fig, and corn. The most stable and the most sensitive peroxidase for spectrophotometric assay is that from horseradish, thus horseradish peroxidase is recommended to be used.

(iV) Assay method

Following is the procedure of the ELISA by using the conjugate described above. First, solid phase (polystylene bead etc.) adsorbed with the antibody is reacted with the Antigen (bacterial cell culture or specimen from patients such as diarrheal stool) After washing the solid phase, the conjugate described above is added and the antibody conjugated with the peroxidase reacts with antigen that has been associated with the antibody on the solid phase, thus forming antibody-antigen-antibody (sandwich) complex. Next, the solid phase is again washed and then peroxidase activity remaining on the solid phase through binding the antibody is measured. To measure the enzyme activity, a substrate such as tetramethylbenzidine is to be used. After the reaction is terminated with 2 M $H_2SO_4$ solution, for example, the absorbance of the specific color developed is measured at 450 nm, for example. If the relation between the amount of the antigen and absorbance has been determined by using the standard antigene, the amount of the antigen (number of the bacteria) in the sample is calculated from the standard curve.

Experimental results

(a) Preparation of reagent for assay

Horseradish peroxidase (6 mg) in 1 ml of 0.1 M sodium phosphate buffer (pH 7.0) was incubated with 0.1 ml of 37.5 mM N-($\epsilon$- maleimidocaproyloxy)-succimide at 30°C for 30-60 min. The mixture was then applied to a column of Sephadex G-25. Material was eluted and the fractions containing horseradish peroxidase with maleimide group were collected, and concentrated.

On the other hand, IgG of polyclonal antibody or monoclonal antibody against bacteria was incubated with pepsin at 37° for 15 hours and anti-bacteria-F(ab′)$_2$ was obtained. Anti-bacteria-F(ab′)$_2$ was reduced with 2- mercaptoethylamine for 90 min at 37°C and the mixture was applied to a column of Sephadex G-25. Material was eluted and the fractions containing Fab′ were collected and concentrated.

Concentrated horseradish peroxidase with a maleimide group and anti-bacteria-Fab′ were incubated together, at moler ratio 1:1, at 30°C for 60 min (or at 4°C, 20 hours) and applied on a column of Ultrogel AcA 44 to separate enzyme labeled antibodies, free enzymes, and antibodies. The fractions containing enzyme-labeled antibodies were collected and used for the assay.

(b) Enzyme-linked immunosorbent assay for Escherichia coli using polyclonal anti-Escherichia coli-reagent

E. coli was cultured in a broth for 18-24 hours at 37° C and was collected by centrifugation. Collected E. coli was suspended in 10 mM sodium phosphate buffer (pH 7.0) containing 0.1% bovine serum albumin, 0.1% sodium azide and 0.1 M NaCl. Standard solutions (0.5 ml each) with different numbers of E. coli were prepared.

Polyclonal anti-E. coli-IgG coated on polysty rene beads was put into each standard solution and incubated at 37° C for 1 hour. Incubated beads were washed with 10 mM sodium phosphate buffer (pH 7.0) containing 0.1 M NaCl. After washing, beads were incubated with enzyme-labeled-anti-E. coli reagent at 37° C for 1 hour. After washing with the same buffer, beads were incubated with tetramethylbenzidine as substrate at 30° C for 1 hour. Enzyme reaction was stopped with 2 M $H_2SO_4$ and the absorbance of the color developed was measured at 450 nm with a spectrophotometer. In this system, as few as 6 x $10^4$ cells/ml of E. coli were detectable (Fig. 1).

(C-1) Enzyme-linked immunosorbent assay for Shigella flexneri using polyclonal anti-Shigella flexneri-reagent

Shigella flexneri was cultured in a broth for 18-24 hours at 37° C and was collected by centrifugation. Collected cells were suspended in 10 mM sodium phosphate buffer (pH 7.0) containing o.1% bovine serum albumin, 0.1% sodium azide and 0.1 M NaCl. Standard solution (0.5 ml each) with different numbers of S. flexneri were prepared.

Polyclonal anti-S. flexneri-IgG coated on polystyrene beads was put into standard solution and incubated at 37° C for 1 hour. Incubated beads were washed with 10 mM sodium phosphate buffer (pH 7.0) containing 0.1 M NaCl. After washing, beads were incubated with enzyme-labeled-anti S. flexneri reagent at 37° C for 1 hour. After washing with the same buffer, beads were incubated with tetramethylbenzidine as substrate at 30° C for 1 hour. Enzyme reaction was stopped with 2 M $H_2SO_4$ and the absorbance of the color developed was measured at 450 nm with a spectrophotometer. In this system, as few as $10^5$ cells/ml of S. flexneri were detectable (Fig. 2).

(C-2) Enzyme-linked immunosorbent assay for Shigella flexneri using monoclonal anti-S. flexneri-reagent

Standard solutions prepared in (C-1) were used in this experiment. Monoclonal anti-S. flexneri-IgG coated on polystyrene beads was put into standard solutions and incubated at 37° C for 1, 3, 5, 24 hours. Incubated beads were washed with 10 mM sodium phosphate buffer (pH 7.0) containing 0.1 M NaCl. After washing, beads were incubated with enzyme-labeled-anti-S. flexneri reagent at 37° C for 1 hour. After washing with the same buffer, beads were incubated with tetramethylbenzidine as substrate at 30° C for 1 hour. Enzyme reaction was stopped with 2 M $H_2SO_4$ and the absorbance of the color developed was measured at 450 nm with a spectrophotometer. In this system, sensitivity of the reaction, that is the number of S. flexneri detected by the reaction depended on the duration fo the first incubation. When the first incubation was less than 5 hours, as few as 0.6 x $10^4$ cells/ml of S. flexneri were detected. When the first incubation was 24 hours, as few as 0.6 x $10^3$ cells/ml of S. flexneri were detected (Fig. 3).

Advantages of the invention

This discovery provides a sensitive, rapid and simple method to detect and identify various bacteria. Following are the major advantages of the invention.

(i) The causative bacteria of certain infectious diseases have so far been identified by first culturing on various media and then identifying by testing several biochemical parameters. Thus it takes at least 48 hours, and in some cases more than 72 hours, to identify the causative bacteria. This invention provides a method to detect causative bacteria directly from the enrichment culture thus to identify bacteria within a much shorter time (at most 10 hours) than currently applied methods.

(ii) The invention provides a method to detect and identify causative bacteria directly from specimen, such as diarrheal stool. The method is quite simple, thus provides a possibility of automation of the whole reaction, which is so far difficult in bacterial detection.

## Claims

1. A highly sensitive, simple and rapid enzyme-linked immunosorbent assay by using antibodies against bacterial antigens conjugated with peroxidase by using N-(maleimidoalkanoyloxy)-succimide of the general formula:

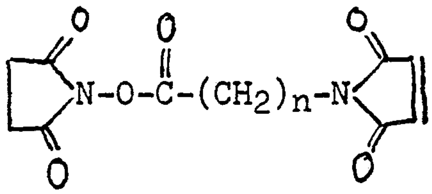

(number of n is 3 to 5)

2. A method as defined in claim 1, for detecting bacteria, in which N- (ε-maleimidocaproyloxy)-succimide (succidyl-6-maleimidohexanoate) is used as N-(maleimidoalkanoyloxy)-succimide.

3. A method as defined in any of claims 1 or 2, in which horseradish peroxidase is used as peroxidase.

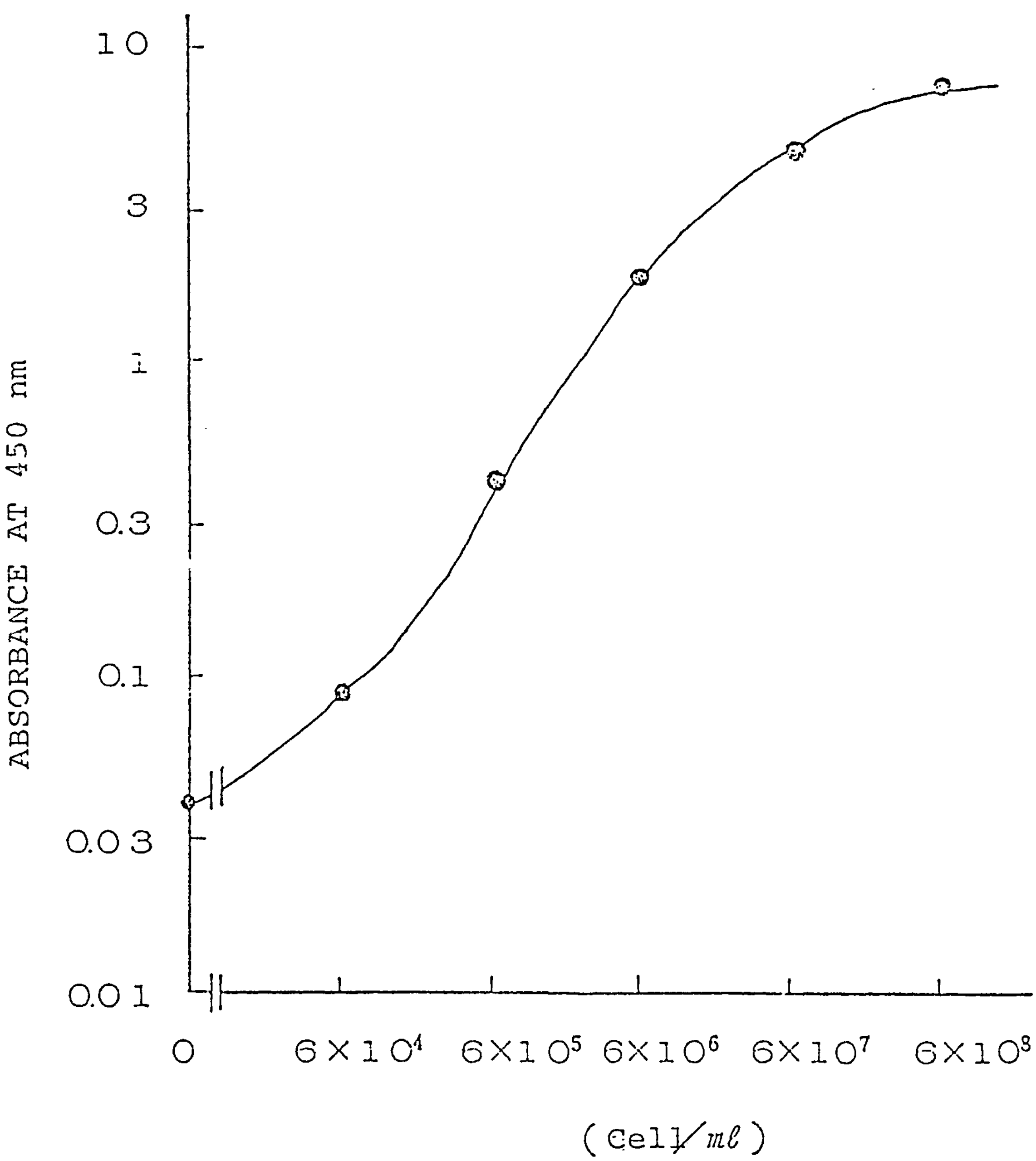
ABSORBANCE AT 450 nm
10
3
1
0.3
0.1
0.03
0.01
0
6×10^4
6×10^5
6×10^6
6×10^7
6×10^8
(Cell/ml)

Fig.1

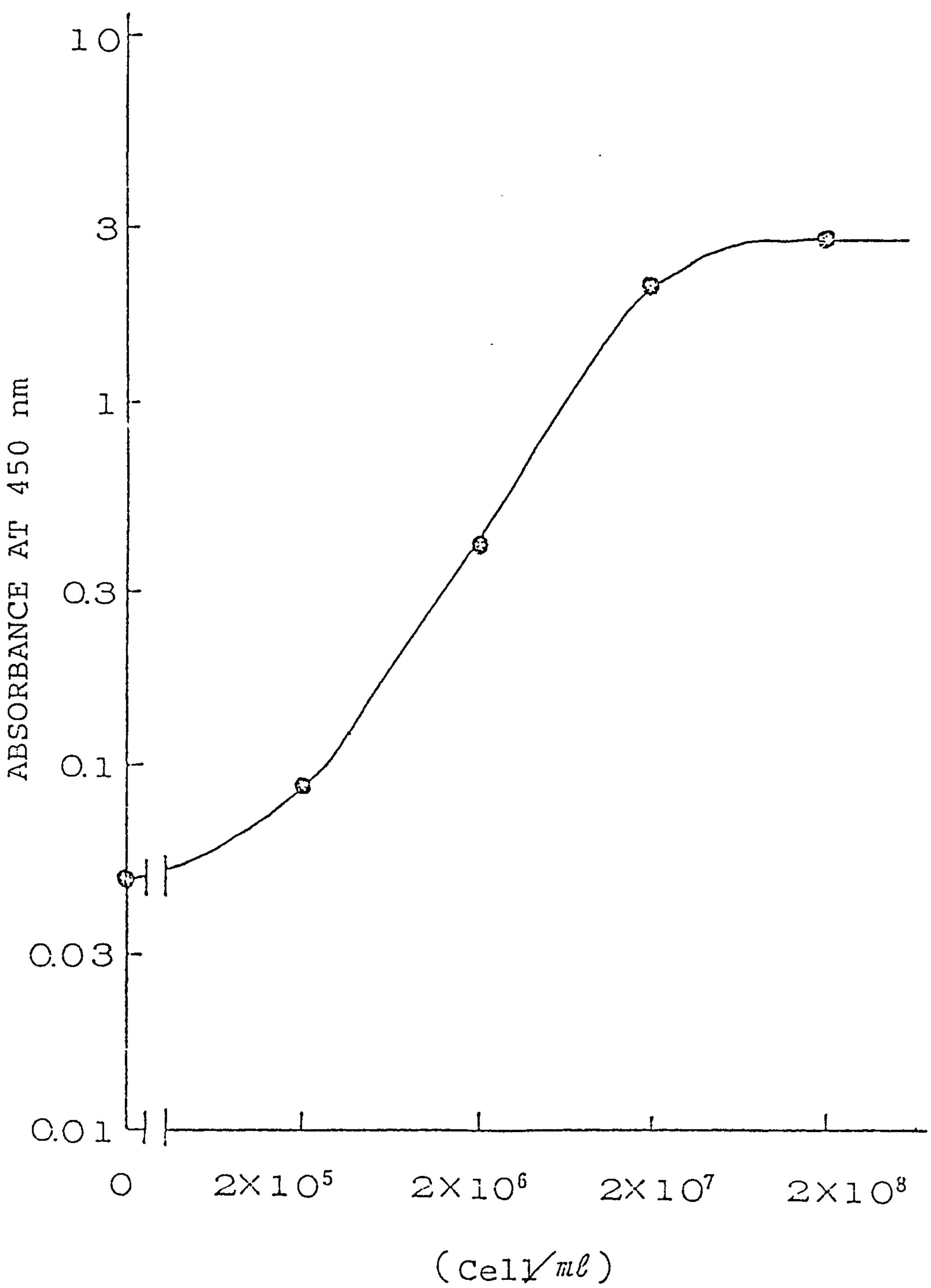
10
3
1
0.3
0.1
0.03
0.01
ABSORBANCE AT 450 nm
0
2×10⁵
2×10⁶
2×10⁷
2×10⁸
(Cell/mℓ)

Fig.2

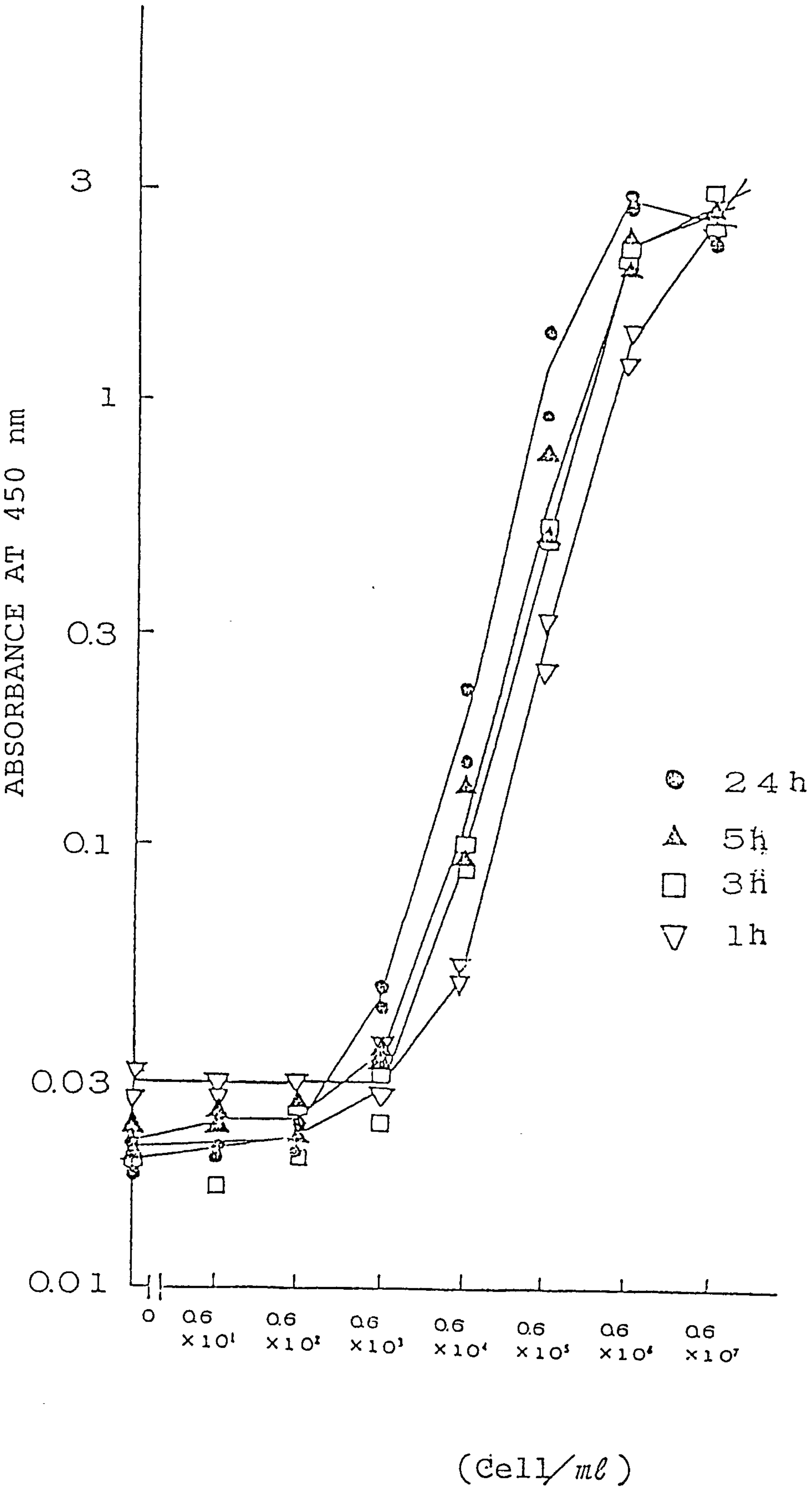
ABSORBANCE AT 450 nm
3
1
0.3
0.1
0.03
0.01
0
0.6 ×10¹
0.6 ×10²
0.6 ×10³
0.6 ×10⁴
0.6 ×10⁵
0.6 ×10⁶
0.6 ×10⁷
24h
5h
3h
1h
(Cell/ml)

Fig.3

European Patent Office

# EUROPEAN SEARCH REPORT

Application Number

EP 88 11 8706

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | EP-A-0 158 291 (TAKEDA CHEMICAL INDUSTRIES, LTD)<br>* Abstract; page 13, line 24 - page 14, line 1; claims 1,4 *<br>--- | 1-3 | G 01 N 33/569<br>G 01 N 33/535 |
| A | EP-A-0 174 106 (BECTON, DICKINSON AND CO.)<br>* Page 3, lines 17-24; page 9, line 12 - page 10, line 18 *<br>--- | 1 | |
| A | EP-A-0 166 164 (E.I. DU PONT DE NEMOURS AND CO.)<br>----- | | |
| | | | TECHNICAL FIELDS SEARCHED (Int. Cl.4) |
| | | | G 01 N |

The present search report has been drawn up for all claims

| Place of search | Date of completion of the search | Examiner |
|---|---|---|
| THE HAGUE | 03-03-1989 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)